# EUROPEAN PATENT SPECIFICATION

(11) **EP 2 200 655 B1**
(45) Date of publication and mention of the grant of the patent: **18.07.2012**
(21) Application number: 08805209.7
(22) Date of filing: 10.10.2008
(51) Int. Cl.: A61K 49/04, C07C 237/46

(54) **CONTRAST AGENTS**
KONTRASTMITTEL
PRODUITS DE CONTRASTE

(30) Priority: 12.10.2007 NO 20075257; 30.11.2007 NO 20076193
(43) Date of publication of application: 30.06.2010
(73) Proprietor: GE Healthcare AS, 0401 Oslo (NO)
(72) Inventor: WYNN, Duncan, George, Amersham, Buckinghamshire HP7 9LL (GB); WISTRAND, Lars-Göran, S-22460 Lund (SE)
(74) Representative: Wulff, Marianne Weiby
(86) International application number: PCT/EP2008/063588
(87) International publication number: WO 2009/047318

(56) References cited:
- EP-A- 0 023 992
- WO-A-92/08691

## Description

### Technical Field of the Invention

The present invention relates to a class of compounds and to diagnostic compositions containing such compounds where the compounds are iodine containing compounds. More specifically the iodine containing compounds are chemical compounds containing two linked iodinated phenyl groups.

The invention also relates to such diagnostic compositions for use in diagnostic imaging as contrast agents and in particular in X-ray imaging, and to contrast media containing such compounds.

### Description of Related art

All diagnostic imaging is based on the achievement of different signal levels from different structures within the body. Thus in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. Moreover, the greater the contrast the smaller the body structures that may be visualized in the imaging procedures, i.e. increased contrast can lead to increased spatial resolution.

The diagnostic quality of images is strongly dependent on the inherent noise level in the imaging procedure, and the ratio of the contrast level to the noise level can thus be seen to represent an effective diagnostic quality factor for diagnostic images.

Achieving improvement in such a diagnostic quality factor has long been and still remains an important goal. In techniques such as X-ray, magnetic resonance imaging (MRI) and ultrasound, one approach to improving the diagnostic quality factor has been to introduce contrast enhancing materials formulated as contrast media into the body region being imaged.

Thus in X-ray early examples of contrast agents were insoluble inorganic barium salts which enhanced X-ray attenuation in the body zones into which they distributed. For the last 50 years the field of X-ray contrast agents has been dominated by soluble iodine containing compounds. Commercial available contrast media containing iodinated contrast agents are usually classified as ionic monomers such as diatrizoate (marketed e.g. under the trade name Gastrografen™), ionic dimers such as ioxaglate (marketed e.g. under the trade name Hexabrix™), nonionic monomers such as iohexol (marketed e.g. under the trade name Omnipaque™), iopamidol (marketed e.g. under the trade name Isovue™), iomeprol (marketed e.g. under the trade name Iomeron™) and the non-ionic dimer iodixanol (marketed under the trade name and Visipaque™).

The most widely used commercial non-ionic X-ray contrast agents such as those mentioned above are considered safe. Contrast media containing iodinated contrast agents are used in more that 20 millions of X-ray examinations annually in the USA and the number of adverse reactions is considered acceptable. However, since a contrast enhanced X-ray examination will require up to about 200 ml contrast media administered in a total dose, there is a continuous drive to provide improved contrast media.

The utility of the contrast media is governed largely by its toxicity, by its diagnostic efficacy, by adverse effects it may have on the subject to which the contrast medium is administered, and by the ease of storage and ease of administration. Since such media are conventionally used for diagnostic purposes rather than to achieve direct therapeutic effect, it is generally desirable to provide media having as little as possible effect on the various biological mechanisms of the cells or the body as this will lead to lower toxicity and lower adverse clinical effect. The toxicity and adverse biological effects of a contrast medium are contributed to by the components of the formulation medium, e.g. the solvent or carrier as well as the contrast agent itself and its components such as ions for the ionic contrast agents and also by its metabolites.

The major contributing factors to the toxicity of the contrast medium are identified as the chemotoxicity of the contrast agent, the osmolality of the contrast medium and the ionic composition or lack thereof of the contrast medium.

Desirable characteristics of an iodinated contrast agent are low toxicity of the compound itself (chemotoxicity), low viscosity of the contrast medium wherein the compound is dissolved, low osmolality of the contrast medium and a high iodine content (frequently measured in g iodine per ml of the formulated contrast medium for administration). The iodinated contrast agent must also be completely soluble in the formulation medium, usually an aqueous medium, and remain in solution during storage.

The osmolalities of the commercial products, and in particular of the non-ionic compounds is acceptable for most media containing dimers and non-ionic monomers although there is still room for improvement. In coronary angiography for example, injection into the circulatory system of a bolus dose of contrast medium has caused severe side effects. In this procedure contrast medium rather than blood flows through the system for a short period of time, and differences in the chemical and physiochemical nature of the contrast medium and the blood that it replaces can cause undesirable adverse effects such as arrhythmias, QT prolongation and reduction in cardiac contractive force. Such effects are seen in particular with ionic contrast agents where osmotoxic effects are associated with hypertonicity of the injected contrast medium. Contrast media that are isotonic or slightly hypotonic with the body fluids are particularly desired. Low osmolar contrast media have low renal toxicity which is particularly desirable. The osmolality is a function of the number of particles per volume unit of the formulated contrast medium.

In patients with acute renal failure, nephropathy induced by contrast medium remains one of the most clinically important complications of the use of iodinated contrast medium. Aspelin, P et al, The New England Journal of Medicine, Vol. 348:491-499 (2003) concluded that nephropathy induced by contrast medium may be less likely to develop in high risk patients when iodixanol is used rather than a low-osmolar, non-ionic contrast medium.

The part of the patient population considered as high risk patients is increasing. To meet the need for continuous improvement of in vivo X-ray diagnostic agents for the entire patient population, there is a continuous drive in finding X-ray contrast agents that has improved properties, also with regards to contrast induced nephrotoxicity (CIN).

To keep the injection volume of the contrast media as low as possible it is highly desirable to formulate contrast media with high concentration of iodine/ml, and still maintain the osmolality of the media at a low level, preferably below or close to isotonicity. The development of non-ionic monomeric contrast agents and in particular non-ionic bis(triiodophenyl) dimers such as iodixanol (EP patent 108638) has provided contrast media with reduced osmotoxicity allowing contrast effective iodine concentration to be achieved with hypotonic solution, and has even allowed correction of ionic imbalance by inclusion of plasma ions while still maintaining the contrast medium Visipaque™ at the desired osmolality (WO 90/01194 and WO 91/13636).

The X-ray contrast media at commercial high iodine concentration have relative high viscosity, ranging from about 15 to about 60 mPas at ambient temperature. Generally, contrast media where the contrast enhancing agent is a dimer has higher viscosity than the corresponding contrast media where the contrast enhancing agent is the monomer corresponding to the dimer. Such high viscosities may pose problems to the administrators of the contrast medium, requiring relatively large bore needles or high applied pressure, and are particularly pronounced in pediatric radiography and in radiographic techniques which require rapid bolus administration, e.g. in angiography.

X-ray contrast media containing a chemical compound as the active pharmaceutical ingredient(s) having two triiodinated phenyl groups linked by a linking group are usually referred to as dimeric contrast agents or dimers. During the years a wide variety of iodinated dimers have been proposed. Relevant patent publications comprises EP 1186305, EP 686046, EP108638, EP 0049745, EP 0023992, WO 2003080554, WO2000026179, WO 1997000240, WO 9208691, US3804892, US4239747, US3763226, US3763227 and US3678152. At this time, one contrast medium having an iodinated non-ionic dimer as the active pharmaceutical ingredient is one the market, the product Visipaque™ containing the compound iodixanol. The compound Hexabrix™, containing the ionic dimeric compound ioxaglic acid is also on the market.

WO92/08691 of Dibra and Bracco proposes symmetrical or asymmetrical 1,3-bis-[3-(mono- or poly-hydroxy)acylamino-5-(mono- or poly-hydroxyalkyl)aminocarbonyl-2,4,6-triiodo-benzoyl-amino]-hydroxy or hydroxyalkyl-propanes and exemplifies a number of these compounds. Tables 1 and 2 provide some test results of the compounds of Examples 1 and 10 of the patent specification. However, none of the compounds prepared in WO92/08691 are developed and brought to the market.

Hence there still exists a desire to develop contrast agents that solves one or more of the problems discussed above. Such agents should ideally have improved properties over the soluble iodine containing compounds on the market in one or more of the following properties: renal toxicity, osmolality, viscosity, solubility, injection volumes/iodine concentration and attenuation/radiation dose and any additional adverse effect known or discovered for such iodinated compounds.

### Summary of the Invention

The present invention provides compounds useful as contrast media having desired properties with regards to at least one of the criteria mentioned above and in particular to renal toxicity, osmolality, viscosity and solubility. The contrast media comprises iodine containing contrast enhancing compounds where iodine containing compounds are chemical compounds containing two linked iodinated phenyl groups. The iodine containing contrast enhancing compounds can be synthesized from commercially available and relatively inexpensive starting materials.

### Detailed Description of the Invention

The new compounds of the invention, their use as X-ray contrast agents, their formulation and production are specified in the attached claims and in the specification hereinafter.

The contrast enhancing compounds are synthetic chemical compounds of formula (I) 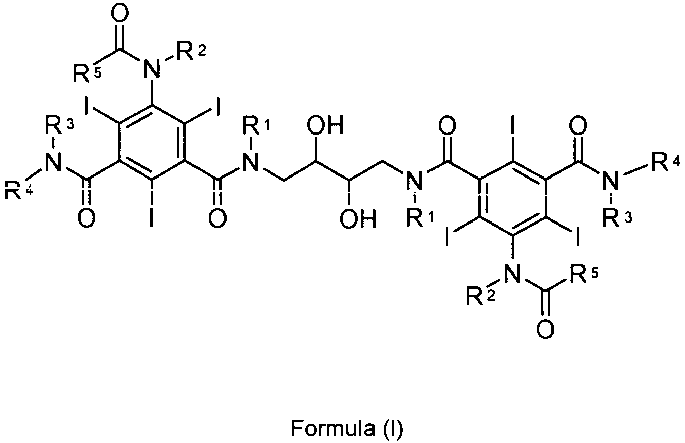 and salts or optical active isomers thereof ,
wherein
each R¹ independently are the same or different and denotes a hydrogen atom or a C₁ to C₄ straight or branched alkyl group;
each R² independently are the same or different and denotes a hydrogen atom or a C₁ to C₄ straight or branched alkyl group;
each R³ independently are the same or different and denotes a hydrogen atom or a C₁ to C₄ straight or branched alkyl group;
each R⁴ independently are the same or different and denote C₁ to C₆ straight or branched alkyl moieties substituted by up to 6 -OH groups; and each R⁵ independently are the same or different and denote C₁ to C₆ straight or branched alkyl moieties substituted by up to 6 -OH groups.

In formula (I) above, the R¹, R² and R³ groups each preferably denote a hydrogen atom and/or a methyl group. Further, each of the R¹ groups are preferably the same, each of the R² groups are preferably the same, and each of the R³ groups are preferably also the same. Most preferred each of the R¹ groups denotes a methyl group, each of the R² groups preferably denotes a hydrogen atom and each of the R³ groups preferably denotes a hydrogen atom or methyl group.

The substituents R⁴ each preferably denote a mono-, di- and tri-hydroxylated C₁ to C₆ straight chain alkyl group. It is further preferred that the alkyl groups carry a hydroxyl group in the ω position and that the alkyl chain is not substituted in the α position. More preferred R⁴ denotes mono- or di hydroxylated propyl moieties and/or hydroxyethyl moieties. Still more preferred each R⁴ group is also the same, and most preferably denotes a 2,3 di-hydroxypropyl moieties.

The substituent R⁵ preferably denotes a di- and tri-hydroxylated C₁ to C₆ straight chain alkyl group. It is further preferred that the alkyl groups carry a hydroxyl group in the ω position. More preferred R⁵ are di-hydroxy-ethyl and/or hydroxymethyl moieties. Still more preferred R⁵ are the same and are 1,2-dihydroxyethyl or hydroxymethyl moieties

Thus, preferred structures according to the invention include the compounds of formula (IIa) to (IId):

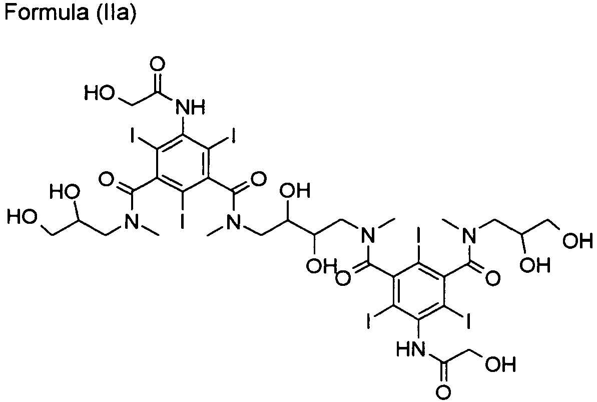

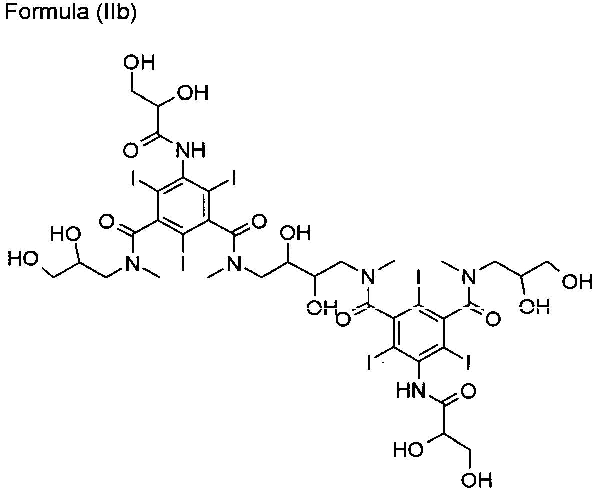

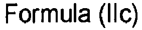

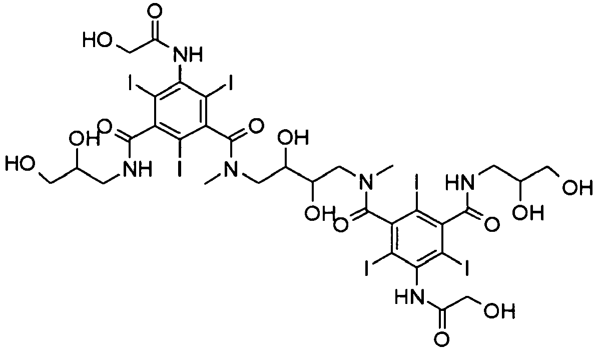

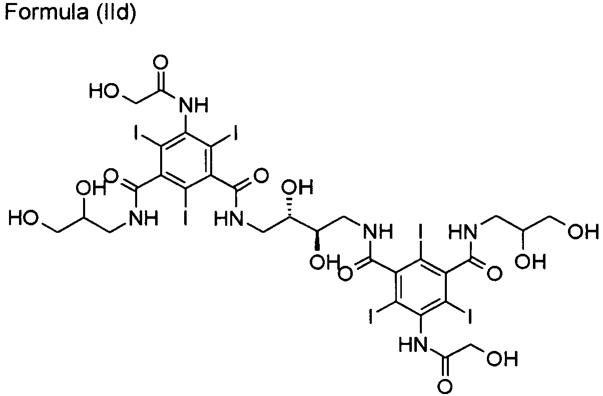

At an iodine concentration of 320 mg/ml, which is a common concentration for commercially available iodinated contrast media, the concentration of the compound of formula (I) will be approximately 0.42 M (Molar). The contrast medium will also be hypoosmolar at this iodine concentration, and this is an advantageous property with regards to the nephrotoxicity of the contrast medium. It is also possible to add electrolytes to the contrast medium to lower the cardiovascular effects as explained in WO 90/01194 and WO 91/13636.

Compounds of formula (I) also comprises optical active isomers and may exist in several isomeric forms due to chiral carbon atoms. In addition, the compounds exhibit exo/endo isomerism due to the restricted rotation of the amide bond caused by the proximity of the bulk iodine atom. Both enantiomerically pure products as well as mixtures of optical isomers are included.

The compounds of the invention may be used as contrast agents and may be formulated with conventional carriers and excipients to produce diagnostic contrast media.

Thus viewed from a further aspect the invention provides a diagnostic composition comprising a compound of formula (I) as described above together with at least one physiologically tolerable carrier or excipient, e.g. in aqueous solution for injection optionally together with added plasma ions or dissolved oxygen.

The contrast agent composition of the invention may be in a ready to use concentration or may be a concentrate form for dilution prior to administration. Generally compositions in a ready to use form will have iodine concentrations of at least 100 mg I/ml, preferably at least 150 mg I/ml, with concentrations of at least 300 mg I/ml, e.g. 320 mg I/ml being preferred. The higher the iodine concentration, the higher is the diagnostic value in the form of X-ray attenuation of the contrast media. However, the higher the iodine concentration the higher is the viscosity and the osmolality of the composition. Normally the maximum iodine concentration for a given contrast media will be determined by the solubility of the contrast enhancing agent, e.g. the iodinated compound, and the tolerable limits for viscosity and osmolality.

For contrast media which are administered by injection or infusion, the desired upper limit for the solution's viscosity at ambient temperature (20°C) is about 30 mPas, however viscosities of up to 50 to 60 mPas and even more than 60 mPas can be tolerated. For contrast media given by bolus injection, e.g. in angiographic procedures, osmotoxic effects must be considered and preferably the osmolality should be below 1 Osm/kg H₂O, preferably below 850 mOsm/kg H₂O, and more preferably about 300 mOsm/kg H₂O.

With the compounds of the invention such viscosity, osmolality and iodine concentrations targets can be met. Indeed, effective iodine concentrations can be reached with hypotonic solutions. It may thus be desirable to make up the solution's tonicity by the addition of plasma cations so as to reduce the toxicity contribution that derives from the imbalance effects following bolus injection. Such cations will desirably be included in the ranges suggested in WO 90/01194 and WO 91/13636.

In particular, addition of sodium and calcium ions to provide a contrast medium isotonic with blood for all iodine concentrations is desirable and obtainable. The plasma cations may be provided in the form of salts with physiologically tolerable counterions, e.g. chloride, sulphate, phosphate, hydrogen carbonate etc., with plasma anions preferably being used.

The contrast media containing compounds of formula (I) can be administered by injection or infusion, e.g. by intervascular administration. Alternatively, contrast media containing compounds of formula (I) may also be administered orally. For oral administration the contrast medium may be in the form of a capsule, tablet or as liquid solution.

In a further embodiment the invention provides diagnostic agents comprising a compound of formula (I) and diagnostic compositions comprising a compound of formula (I) together with pharmaceutically acceptable carriers or excipients. The diagnostic agents and composition are preferably for use in X-ray diagnosis.

Hence, the invention further embraces use of a diagnostic agent and a diagnostic composition containing a compound of formula (I) in X-ray contrast examinations and use of a compound of formula (I) for the manufacture of a diagnostic composition for use as an X-ray contrast agent.

Provided are compounds of formula (I) for use in a method of diagnosis comprising administration of compounds of formula (I) to the human or animal body, examining the body with a diagnostic device and compiling data from the examination. In said method of diagnosis the body may also be preadministrated with compounds of formula (I).

Furthermore, a method of imaging, specifically X-ray imaging is provided, which comprises administration of compounds of formula (I) to the human or animal body, examining the body with a diagnostic device and compiling data from the examination and optionally analysing the data. In the method of imaging the body may also be preadministrated with compounds of formula (I).

### Preparation

The compounds of the general formula (I) can be synthesized by multistep procedures from starting materials that are either known from the state of art or that are commercially available or can readily be produced from commercially available materials.

Compounds of formula (I) can be synthesized according to this general procedure: 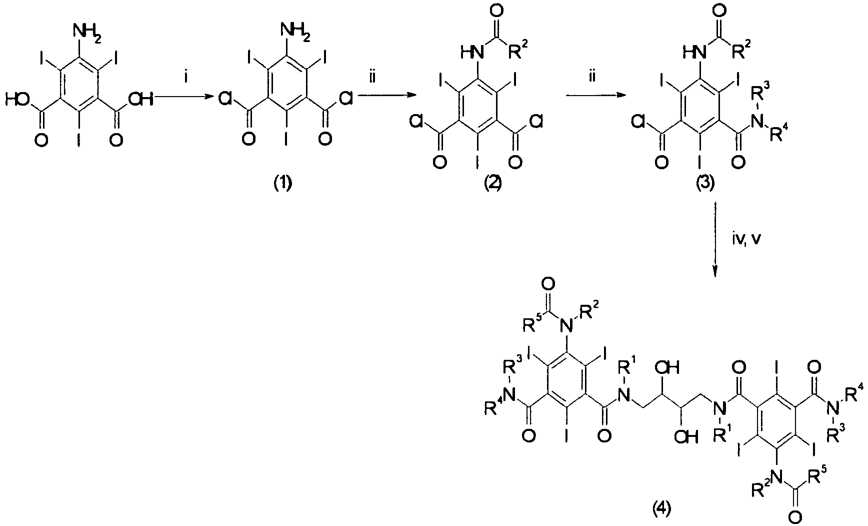 (i) SOCl₂, pyr, DCM, 70°C; (ii) R⁵COCl, DMAc; (iii) R³NHR⁴, NEt₃, DMAc; (iv) R¹NHCH₂CHOHCHOHCH₂NH R¹, NEt₃, DMAc; (v) NH₃, MeOH

5-amino-2,4,6-triiodo-isophtalic acid available from Aldrich is treated with thionyl chloride to form the corresponding 5-amino-2,4,6-triiodo-isophthaloyl dichloride (1). 5-Amino-2,4,6-triiodo-isophthaloyl dichloride is next reacted with either acetoxyacetyl chloride commercially available from Aldrich to form the desired N-acyl derivatives (2). N-acyl-amino-2,4,6-triiodo-isophthaloyl dichloride is then reacted with an appropriate amine such as 3-amino-1,2-propanediol to form the desired mono-amide derivatives (3). The dimer (4) is finally formed by reacting with an appropriate di-amine such as 1,4 -diaminobutane-2,3-diol with the desired mono-amide (3), follow by hydrolysis of the protecting groups.

1,4 -diamino-butane-2,3-diol HCl is commercially available, e.g from Fluka.

### Preparation of intermediates:

### Preparation A

### Acetic acid (3,5-bis-chlorocarbonyl-2,4,6-triiodo-phenylcarbamoyl)-methyl ester

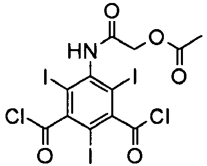

5-Amino-2,4,6-triiodo-isophthaloyl dichloride was dissolved in dimethyl acetamide (DAMc) and a solution of acetoxyacetylchloride (2eq) in DMAc was slowly added with efficient stirring. The reaction mixture was stirred overnight and the following day, the mixture was slowly poured into stirred ice water. The precipitate was filtered off and dried to give the desired material. The structure was confirmed by ¹H NMR (CDCl₃, 300MHz) : 10.43 (br s, 1H); 4.71 (s, 2H); 2.11 (s, 3H)

### Preparation B

### Acetic acid 2-acetoxy-1-(3,5-bis-chlorocarbonyl-2,4,6-triiodo-phenylcarbamoyl)-ethyl ester

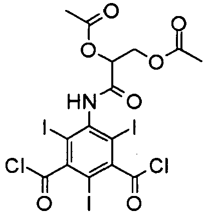

The structure was confirmed by ¹H NMR (CDCl₃, 300MHz) : 10.45 (br s, 1H); 4.49-4.30 (m, 3H); 2.13 (s, 3H).

Following this procedure various compounds of formula (2) above can be prepared, including

### Acetic acid 2,3-diacetoxy-1-(3,5-bis-chlorocarbonyl-2,4,6-triiodo-phenylcarbamoyl)-propyl ester

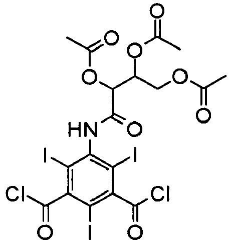

### Preparation C

### Acetic acid {3-chlorocarbonyl-5-[(2,3-dihydroxy-propyl)-methyl-carbamoyl]-2,4,6-triiodo-phenylcarbamoyl}-methyl ester

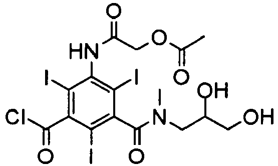

The bis-acid chloride from the previous step was dissolved in DMAC in a dry flask under a nitrogen atmosphere. Triethylamine (2eq) was added to the solution immediately followed by the addition of 3-Methylamino-propane-1,2-diol (2eq). After stirring overnight, the reaction mixture was concentrated to dryness, and the residue purified by chromatography using silica gel to give the desired product. The structure was confirmed by ¹H NMR (DMSO-D6, 300MHz) : 10.4 (br s, 1 H); 4.70 (s, 2H); 3.89-3.83 (m, 1 H); 3.75-3.67 (m, 1 H); 3.51-3.42 (m, 2H); 3.25-3.15 (m, 1 H); 2.85 (s, 3H); 2.15 (s, 3H)

### Preparation D

### Acetic acid 2-acetoxy-1-{3-chlorocarbonyl-5-[(2,3-dihydroxy-propyl)-methylcarbamoyl]-2,4,6-triiodo-phenylcarbamoyl}-ethyl ester

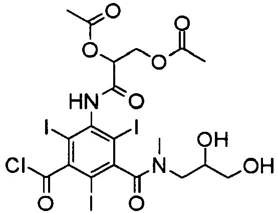

The structure was confirmed by ¹H NMR (DMSO-D6, 300MHz) : 10.4 (br s, 1H); 4.70-4.65 (m, 3H); 3.89-3.83 (m, 1 H); 3.75-3.67 (m, 1 H); 3.51-3.42 (m, 2H); 3.25-3.15 (m, 1 H); 2.85 (s, 3H); 2.15 (s, 3H).

### Preparation E

### Acetic acid 2,3-diacetoxy-1-{3-chlorocarbonyl-5-[(2,3-dihydroxy-propyl)-methylcarbamoyl]-2,4,6-triiodo-phenylcarbamoyl}-propyl ester

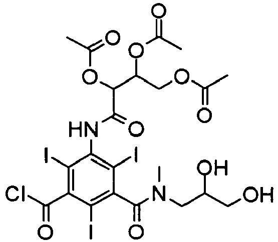

The structure was confirmed by NMR and MS.

Following this procedure various compounds of formula (3) above can be prepared, including :

### Acetic acid [3-chlorocarbonyl-5-(2,3-dihydroxy-propylcarbamoyl)-2,4,6-triiodo-phenylcarbamoyl]-methyl ester

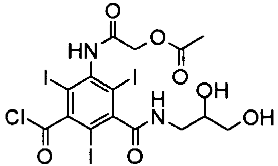

### Acetic acid 2-acetoxy-1-[3-chlorocarbonyl-5-(2,3-dihydroxy-propylcarbamoyl)-2,4,6-triiodo-henylcarbamoyl]-ethyl ester

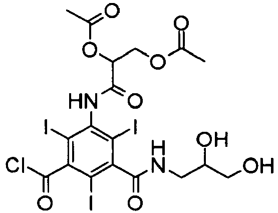

### Acetic acid 2,3-diacetoxy-1-[3-chlorocarbonyl-5-(2,3-dihydroxy-propylcarbamoyl)-2,4,6-triiodo-phenylcarbamoyl]-propyl ester

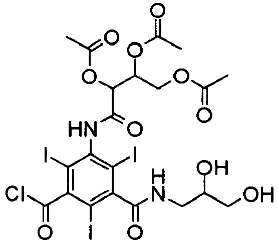

### Acetic acid {3-[bis-(2,3-dihydroxy-propyl)-carbamoyl]-5-chlorocarbonyl-2,4,6-triiodo-phenylcarbamoyl}-methyl ester

### Preparation F

Acetic acid (3,5-bis-chlorocarbonyl-2,4,6-triiodo-phenylcarbamoyl)-methyl ester (20g, 25.5mmol) was dissolved in dry DMA (100ml) and 2,2,-dimethyl-1,3-dioxolane-4-methanaine (6.62ml, 51 mmol) was added. The reaction was stirred for 24 hours at room temperature under nitrogen. The reaction mixture was diluted with ethyl acetate and washed with ice-water (50ml x 3) and brine. The organics were collected, dried over MgSO₄, filtered and evaporated to give as brown oil. This was purified by silica column chromatography eluting with petrol: ethyl acetate to give acetic acid {3-chlorocarbonyl-5-[(2,2-dimethyl-[1,3]dioxolan-4-ylmethyl)-carbamoyl]-2,4,6-triiodo-phenylcarbamoyl}-methyl ester (13.85g, 17.5mmol) as a pink solid.
Following this procedure various compounds of formula (3) above can be prepared, including:

### Acetic acid {3-chlorocarbonyl-5-[(2,2-dimethyl-[1,3]dioxolan-4-ylmethyl)-carbamoyl]-2,4,6-triiodo-phenylcarbamoyl}-methyl ester

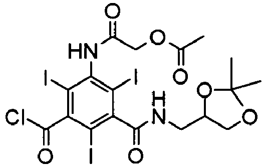

The structure was confirmed by Mass Spec (ESI) m/z: Calculated for C₁₈H₁₈CIN₂O₇ [M+H]⁺ 791.520 Found 790.84, ¹H NMR (DMSO; 300MHz) δ = 10.35-10.15 (m, 1H, NH), 9.03-8.87 (m, 1 H, NH), 4.70 (s, 2H), 4.25(m, 1 H), 4.07 (m, 1 H), 3.79 (m, 1 H), 3.50-3.10 (m, 2H), 2.15 (s, 3H), 1.36 (s, 3H), 1.23 (s, 3H).

### Acetic acid 2-acetoxy-1-[3-chlorocarbonyl-5-(2,3-dihydroxy-propylcarbamoyl)-2,4,6-triiodo-phenylcarbamoyl]-ethyl ester

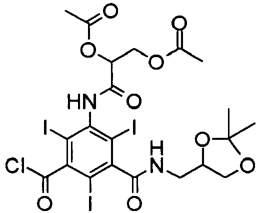

The structure was confirmed by Mass Spec (ESI) m/z: Calculated for C₂₁H₂₂CIN₂O₉ [M+H]⁺ 863.594 Found 862.75, ¹H NMR (CDCl₃; 300MHz) δ = 6.39 (s, br, 1H, NH), 5.63 (s, br, 1 H, NH), 4.64 (m, 1 H), 4.50 (m, 1 H), 4.35 (m, 1 H), 3.78-3.65 (m, 2H), 3.42 (m, 1 H), 2.28 (d, 3H), 2.08 (s, 3H), (s, 3H), 1.43 (s, 3H), 1.33 (s, 3H)

### Example 1

### N¹,N^{1'}-(2,3-dihydroxybutane-1,4-diyl)bis(N³-(2,3-dihydroxypropyl)-5-(2-hydroxyacetamido)-2,4,6-triiodo-N¹,N³-dimethylisophthalamide)

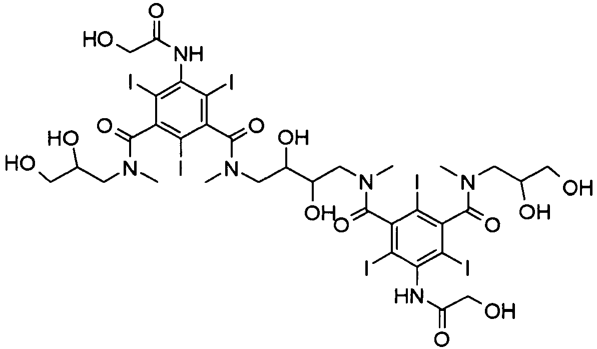

1,4-dimethylamine-2,3-butanediol (0.5 eq) and triethylamine (1.2eq) were added to a solution of acetic acid {3-chlorocarbonyl-5-[(2,3-dihydroxy-propyl)-methylcarbamoyl]-2,4,6-triiodo-phenylcarbamoyl}-methyl ester (2.5g,3.27mmol) in DMA (5mL). The reaction was stirred at ambient temperature until the reaction proceeds no further. The reaction mixture was extracted into ethyl actetate and washed with water to remove the DMA. The organic layer was dried over MgSO4 and the filtrate concentrated under vacuum to give the desired compound which was used in the next step without purification. The crude material was dissolved in the minimum amount of methanol and treated with aqueous ammonia. The reaction was stirred at ambient temperature and monitored by LC-MS. Whereupon, the reaction mixture was concentrated to dryness, dissolved in the minimum amount of water, filtered and purified by preparative HPLC to give the desired final product. Full deprotection could also be carried out by refluxing for 1 hour, the crude mixture in 2M aqueous HCl.

The structure was confirmed by Mass Spec (ESI) m/z: Calculated for C₃₄H₄₂I₆N₆O₁₄ [M]⁺ 1520.13 Found 1520.45

### Example 2

### N¹,N^{1'}-(2,3-dihydroxybutane-1,4-diyl)bis(5-(2,3-dihydroxypropanamido)-N³-(2,3-dihydroxypropyl)-2,4,6-triiodo-N¹,N³-dimethylisophthalamide)

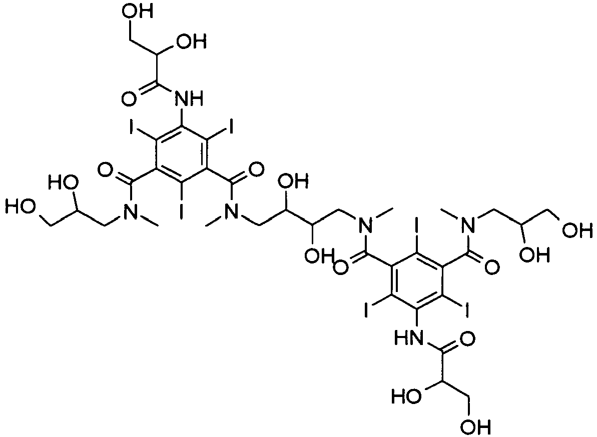

1,4-dimethylamine-2,3-butanediol (0.5 eq) and triethylamine (1.2eq) were added to a solution of Acetic acid 2-acetoxy-1-[3-chlorocarbonyl-5-(2,3-dihydroxy-propylcarbamoyl)-2,4,6-triiodo-phenylcarbamoyl]-ethyl ester (2.5g,2.90 mmol) in DMA (5mL). The reaction was stirred at ambient temperature until the reaction proceeds no further. The reaction mixture was extracted into ethyl actetate and washed with water to remove the DMA. The organic layer was dried over MgSO4 and the filtrate concentrated under vacuum to give the desired compound which was used in the next step without purification. The crude material was dissolved in the minimum amount of methanol and treated with aqueous ammonia. The reaction was stirred at ambient temperature and monitored by LC-MS. Whereupon, the reaction mixture was concentrated to dryness, dissolved in the minimum amount of water, filtered and purified by preparative HPLC to give the desired final product. Full deprotection could also be carried out by refluxing for 1 hour, the crude mixture in 2M aqueous HCl.

The structure was confirmed by Mass Spec (ESI) m/z: Calculated for C₃₆H₄₆I₆N₆O₁₆ [M]⁺ 1580.23 Found 1580.66

### Example 3

### N¹,N^{1'}-(2,3-dihydroxybutane-1,4-diyl)bis(N³-(2,3-dihydroxypropyl)-5-(2-hydroxyacetamido)-2,4,6-triiodo-N¹-methylisophthalamide)

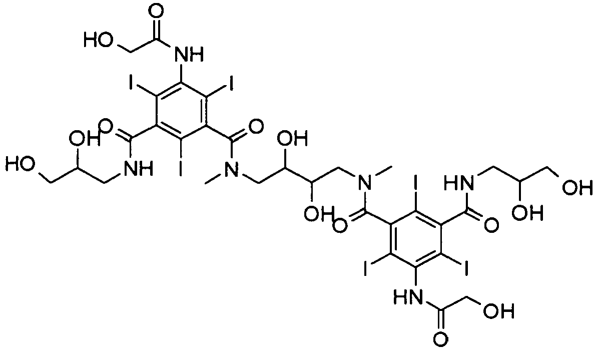

1,4-dimethylamine-2,3-butanediol (0.5eq) and triethylamine (1.2eq) were added to a solution of acetic acid {3-chlorocarbonyl-5[(2,2-dimethyl-[1,3]dioxolan-4-ylmethyl)carbamoyl]-2,4,6-triiodo-phenylcarbamoyl}methyl-ester (2.5g, 3.16mmol) in DMA (5mL). The reaction mixture was stirred at ambient temperature until the reaction proceeds no further. The reaction mixture was extracted into ethyl acetate and washed with water to remove the DMA. The organic layer was dried over MgSO4 and the filtrate concentrated under vacuum to give the desired compound which was used in the next step without purification. Full deprotection was carried out by refluxing for 1 hour, the crude mixture in 1:1 mixture of 2M aqueous HCl and MeOH.

The structure was confirmed by Mass Spec (ESI) m/z: Calculated for C₃₂H38I₆N₆O₁₄ [M]⁺ 1492.02 Found 1492.69

### Example 4

### N¹,N^{1'}-((2R,3S)-2,3-dihydroxybutane-1,4-diyl)bis(N³-(2,3-dihydroxypropyl)-5-(2-hydroxyacetamido)-2,4,6-triiodoisophthalamide)

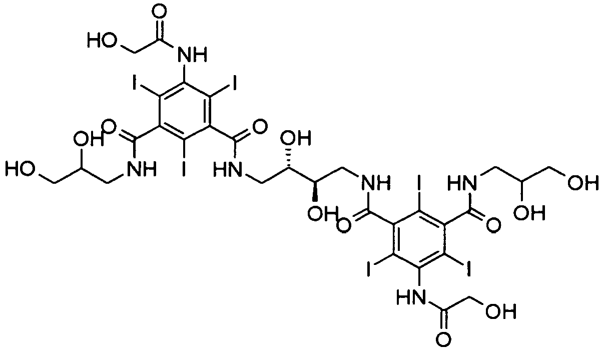

The compound was prepared following the procedure of example 3.

The structure was confirmed by Mass Spec (ESI) m/z: Calculated for C₃₀H₃₄I₆N₆O₁₄ [M]⁺ 1464.03 Found 1464.65

## Claims

1. Compounds of formula (I) 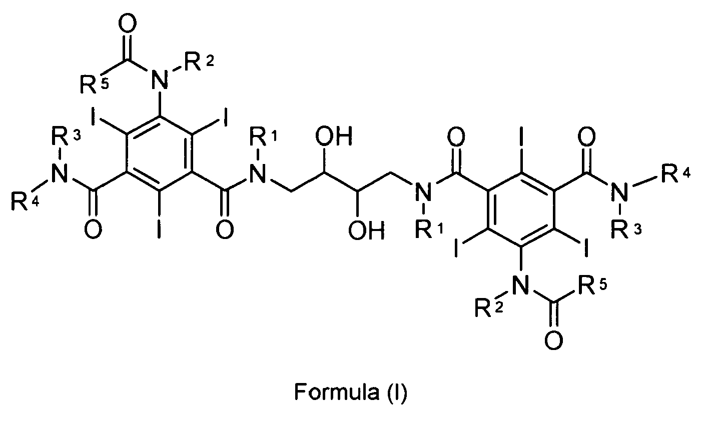 and salts or optical active isomers thereof,
wherein
each of R¹, R² and R³ independently are the same or different and denote a hydrogen atom or a C₁ to C₄ straight or branched alkyl group;
each R⁴ independently are the same or different and denote C₁ to C₆ straight or branched alkyl moieties substituted by up to 6 -OH groups; and
each R⁵ independently are the same or different and denote C₁ to C₆ straight or branched alkyl moieties substituted by up to 6 -OH groups.

2. Compound as claimed in claim 1 wherein each R¹, R² and R³ denote a hydrogen atom and/or a methyl group

3. Compound as claimed in claims 1 or 2 wherein each R¹ denotes a methyl group, each R² denotes a hydrogen atom, and each R³ denotes a hydrogen atom or methyl group.

4. Compound as claimed in any of the preceding claims wherein each R⁴ denotes a mono-, di- and tri-hydroxylated C₁ to C₆ straight chain alkyl group.

5. Compound as claimed in claim 4 wherein R⁴ carry a hydroxyl group in the w position and is not substituted in the α position.

6. Compound as claimed in claims 5 and 6 wherein all R⁴ are the same and are 2,3-dihydroxypropyl moieties.

7. Compounds as claimed in any of the previous claims wherein R⁵ denotes a di- or tri-hydroxylated C₁ to C₆ straight chain alkyl group.

8. Compound as claimed in claim 7 wherein R⁵ are di- or tri hydroxylated propyl moieties and/or di-hydroxy-ethyl moieties.

9. Compound as claimed in claims 7 or 8 wherein each R⁵ are the same and are 1,2-dihydroxyethyl or hydroxymethyl moieties.

10. Compound as claimed in any of the preceding claims selected from the group of
N¹,N^{1'}-(2,3-dihydroxybutane-1,4-diyl)bis(N³-(2,3-dihydroxypropyl)-5-(2-hydroxyacetamido)-2,4,6-triiodo-N¹,N³-dimethylisophthalamide);
N¹,N^{1'}-(2,3-dihydroxybutane-1,4-diyl)bis(5-(2,3-dihydroxypropanamido)-N³-(2,3-dihydroxypropyl)-2,4,6-triiodo-N¹,N³-dimethylisophthalamide);
N¹, N^{1'}-(2,3-dihydroxybutane-1,4-diyl)bis(N³-(2,3-dihydroxypropyl)-5-(2-hydroxyacetamido)-2,4,6-triiodo-N¹-methylisophthalamide); and
N¹,N^{1'}-((2*R*,3*S*)-2,3-dihydroxybutane-1,4-diyl)bis(N³-(2,3-dihydroxypropyl)-5-(2-hydroxyacetamido)-2,4,6-triiodoisophthalamide).

11. An X-ray diagnostic composition comprising a compound of formula (I) as defined in any of the preceding claims together with a pharmaceutically acceptable carriers or excipients.

12. A diagnostic composition containing a compound of formula (I) as defined in any of the preceding claims for use in X-ray contrast examinations.

## Patentansprüche

1. Verbindungen der Formel (I) 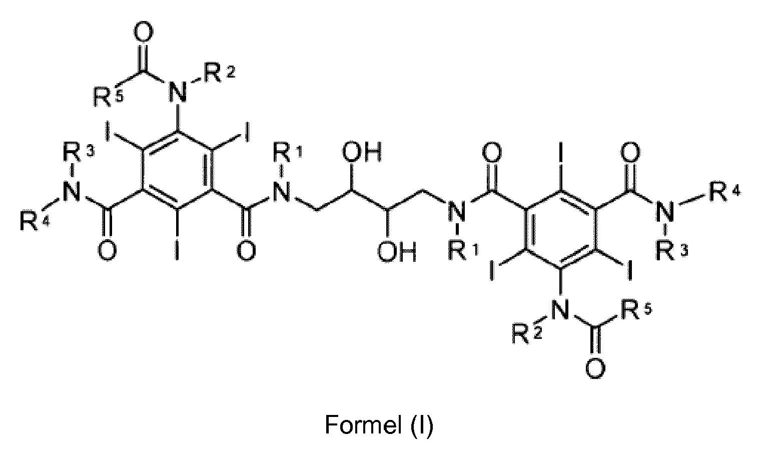 und Salze oder optisch aktive Isomere derselben,
worin
jedes von R¹, R² und R³ unabhängig gleich oder verschieden ist und ein Wasserstoffatom oder eine gerade oder verzweigte C₁- bis C₄-Alkylgruppe bedeutet;
jedes R⁴ unabhängig gleich oder verschieden ist und eine gerade oder verzweigte C₁- bis C₆-Alkyleinheit, substituiert durch bis zu 6 -OH-Gruppen, bedeutet; und
jedes R⁵ unabhängig gleich oder verschieden ist und eine gerade oder verzweigte C₁- bis C₆-Alkyleinheit, substituiert durch bis zu 6 -OH-Gruppen, bedeutet.

2. Verbindung nach Anspruch 1, worin jedes R¹, R² und R³ ein Wasserstoffatom und/oder eine Methylgruppe bedeutet.

3. Verbindung nach Anspruch 1 oder 2, worin jedes R¹ eine Methylgruppe bedeutet, jedes R² ein Wasserstoffatom bedeutet, und jedes R³ ein Wasserstoffatom oder eine Methylgruppe bedeutet.

4. Verbindung nach einem der vorstehenden Ansprüche, worin jedes R⁴ eine mono-, di- und tri-hydroxylierte geradkettige C₁- bis C₆-Alkylgruppe bedeutet.

5. Verbindung nach Anspruch 4, worin R⁴ eine Hydroxygruppe in der ω-Position trägt und in der α-Position nicht substituiert ist.

6. Verbindung nach Ansprüchen 5 und 6, worin alle R⁴ gleich sind und für 2,3-Dihydroxypropyl-Einheiten stehen.

7. Verbindung nach einem der vorstehenden Ansprüche, worin R⁵ eine di- oder tri-hydroxylierte geradkettige C₁- bis C₆-Alkylgruppe bedeutet.

8. Verbindung nach Anspruch 7, worin R⁵ für eine di- oder tri-hydroxylierte Propyleinheit und/oder eine Di-Hydroxy-Ethyl-Einheit steht.

9. Verbindung nach Anspruch 7 oder 8, worin jedes R⁵ gleich ist und für eine 1,2-Dihydroxyethyl- oder Hydroxymethyl-Einheit steht.

10. Verbindung nach einem der vorstehenden Ansprüche, die gewählt ist aus der Gruppe
N¹,N^{1'}-(2,3-Dihydroxybutan-1,4-diyl)bis(N³-(2,3-dihydroxypropyl)-5-(2-hydroxyacetamido)-2,4,6-trijod-N¹,N³-dimethylisophthalamid);
N¹,N^{1'}-(2,3-Dihydroxybutan-1,4-diyl)bis(5-(2,3-dihydroxypropanamido)-N³-(2,3-dihydroxypropyl)-2,4,6-trijod-N¹,N³-dimethylisophthalamid);
N¹,N^{1'}-(2,3-Dihydroxybutan-1,4-diyl)bis(N³-(2,3-dihydroxypropyl)-5-(2-hydroxyacetamido)-2,4,6-trijod-N¹-methylisophthalamid); und
N¹,N^{1'}-((2*R*,3*S*)-2,3-Dihydroxybutan-1,4-diyl)bis(N³-(2,3-dihydroxypropyl)-5-(2-hydroxyacetamido)-2,4,6-trijodisophthalamid).

11. Röntgendiagnostische Zusammensetzung, die eine Verbindung der Formel (I), wie in einem der vorstehenden Ansprüche definiert, zusammen mit pharmazeutisch unbedenklichen Trägern oder Exzipienten umfasst.

12. Diagnostische Zusammensetzung, die eine Verbindung der Formel (I), wie in einem der vorstehenden Ansprüche definiert, zur Verwendung in Röntgenkontrastuntersuchungen enthält.

## Revendications

1. Composés de formule (I) 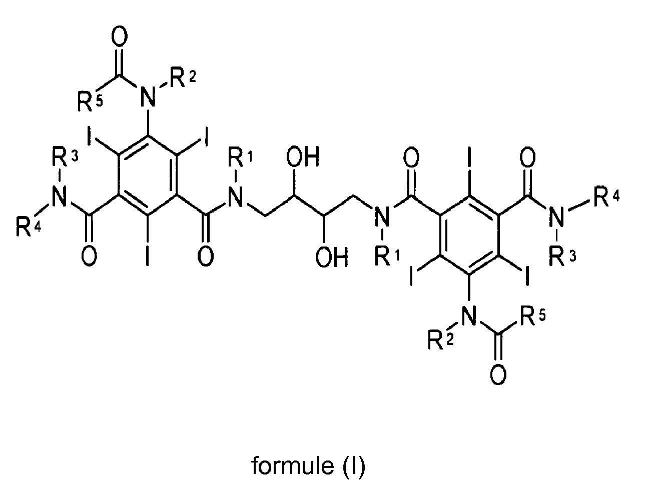 et sels ou isomères optiquement actifs de ceux-ci,
dans laquelle
chacun de R¹, de R² et de R³ est indépendamment identique ou différent et représente un atome d'hydrogène ou un groupe alkyle linéaire ou ramifié en C₁ à C4;
chaque R⁴ est indépendamment identique ou différent et représente des fractions alkyles linéaires ou ramifiés en C₁ à C₆ substituées par jusqu'à 6 groupes -OH ; et
chaque R⁵ est indépendamment identique ou différent et représente des fractions alkyles linéaires ou ramifiés en C₁ à C₆ substituées par jusqu'à 6 groupes -OH.

2. Composé selon la revendication 1 dans lequel chaque R¹, R² et R³ représente un atome d'hydrogène et/ou un groupe méthyle.

3. Composé selon les revendications 1 ou 2 dans lequel chaque R¹ représente un groupe méthyle, chaque R² représente un atome d'hydrogène, et chaque R³ représente un atome d'hydrogène ou un groupe méthyle.

4. Composé selon l'une quelconque des revendications précédentes dans lequel chaque R⁴ représente un groupe alkyle à chaîne linéaire en C₁ à C₆ mono-, di- et tri-hydroxylé.

5. Composé selon la revendication 4 dans lequel R⁴ supporte un groupe hydroxyle en position ω et n'est pas substitué en position α.

6. Composé selon les revendications 5 et 6 dans lequel tous les R⁴ sont identiques et représentent des fractions 2,3-dihydroxypropyle.

7. Composés selon l'une quelconque des revendications précédentes dans lequel R⁵ représente un groupe alkyle à chaîne linéaire en C₁ à C₆ di- ou tri-hydroxylé.

8. Composé selon la revendication 7 dans lequel R⁵ représente des fractions propyles di- ou tri-hydroxylées et/ou des fractions di-hydroxy-éthyles.

9. Composé selon les revendications 7 ou 8 dans lequel chaque R⁵ est identique et représente des fractions 1,2-dihydroxyéthyles ou hydroxyméthyles.

10. Composé selon l'une quelconque des revendications précédentes sélectionné parmi le groupe constitué par :
N¹,N^{1'}-(2,3-dihydroxybutane-1,4-diyl)bis(N³-(2,3-dihydroxypropyl)-5-(2-hydroxyacétamido)-2,4,6-tri-iodo-N¹,N³-diméthylisophthalamide) ;
N¹,N^{1'}-(2,3-dihydroxybutane-1,4-diyl)bis(5-(2,3-dihydroxypropanamido)-N³-(2,3-dihydroxypropyl)-2,4,6-tri-iodo-N¹,N³-diméthylisophthalamide) ;
N ¹,N^{1'}-(2,3-dihydroxybutane-1,4-diyl)bis(N³-(2,3-dihydroxypropyl)-5-(2-hydroxyacétamido)-2,4,6-tri-iodo-N¹-méthylisophthalamide) ; et
N¹,N^{1'}-((2R,3S)-2,3-dihydroxybutane-1,4-diyl)bis(N³-(2,3-dihydroxypropyl)-5-(2-hydroxyacétamido)-2,4,6-tri-iodo-isophthalamide).

11. Composition de diagnostic pour radiographie comprenant un composé de formule (I) selon l'une quelconque des revendications précédentes ainsi que des supports ou excipients pharmaceutiquement acceptables.

12. Composition de diagnostic contenant un composé de formule (I) selon l'une quelconque des revendications précédentes destinée à une utilisation dans des examens de contraste par radiographie.
